# EUROPÄISCHE PATENTSCHRIFT

(11) **EP 0 607 795 B1**
(45) Veröffentlichungstag und Bekanntmachung des Hinweises auf die Patenterteilung: **04.10.1995**
(21) Anmeldenummer: 94100084.6
(22) Anmeldetag: 05.01.1994
(51) Int. Cl.: C07D 277/34

(54) **Verfahren zur Herstellung von 2-Alkyl-4-fluormethyl-thiazolcarbonsäure-alkylestern**
Process for the preparation of 2-alkyl-4-fluoromethyl-thiazole-carboxylic acid esters
Procédé pour la préparation des esters alkyles de l'acide alkyle-2 fluorométhyle-4-thiazole

(30) Priorität: 20.01.1993 DE 4301356
(43) Veröffentlichungstag der Anmeldung: 27.07.1994
(73) Patentinhaber: BASF Aktiengesellschaft, 67063 Ludwigshafen (DE)
(72) Erfinder: Eicken, Karl, Dr., D-67157 Wachenheim (DE); Ditrich, Klaus, Dr., D-66161 Goennheim (DE); Mueller, Thomas, Dr., D-67258 Hessheim (DE); Wagner, Oliver, Dr., D-66450 Bexbach (DE)

(56) Entgegenhaltungen:
- EP-A- 0 276 177
- EP-A- 0 279 239
- EP-A- 0 371 950
- JP-A- 3 127 784
- US-A- 4 045 567

## Beschreibung

Die Erfindung betrifft ein Verfahren zur Herstellung von 2-Alkyl-4-fluormethylthiazolcarbonsäure-alkylestern.

Es ist bekannt, Thioacetamid mit 2-Chlor-4,4,4-trifluormethylacetessigsäureethylester unter längerem Erhitzen auf Temperaturen über 110°C in Dimethylformamid oder Eisessig umzusetzen, wobei man nach destillativer oder säulenchromatographischer Reinigung 2-Methyl-4-trifluormethyl-thiazolcarbonsäureethylester in mäßigen Ausbeuten (38 % bzw. 56 %) erhält (EP 276 177, 371 950).

Es wurde nun gefunden, daß man 2-Alkyl-4-fluormethylthiazolcarbonsäure-alkylester der Formel I 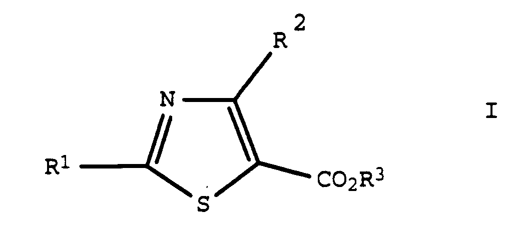
in welcher
- R¹: C₁-C₄-Alkyl
- R²: Difluormethyl oder Trifluormethyl und
- R³: C₁-C₆-Alkyl
bedeuten,
in sehr guten Ausbeuten erhält, wenn man Carbonsäurethioamide der Formel II 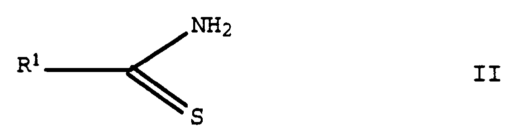
in welcher R¹ die oben angegebene Bedeutung hat, mit 2-Chlor-4-fluoracetessigsäure-alkylestern der Formel III 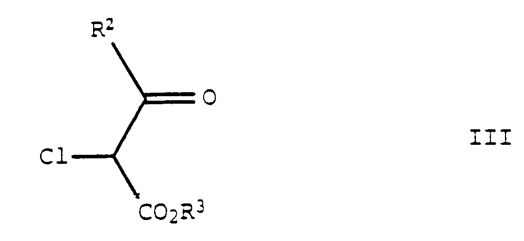
in der R² und R³ die oben angegebene Bedeutung haben, in einem aprotischen Lösungsmittel bei Temperaturen unter 100°C zur Reaktion bringt und mit einem wasserabspaltenden Mittel, gegebenenfalls in Gegenwart von mindestens doppelten bis dreifachen molaren Mengen von Basen, bei Temperaturen unter 80°C umsetzt.

Die Alkylreste bedeuten im einzelnen z.B.
- R¹: Methyl, Ethyl, n-Propyl, i-Propyl, n-Butyl, sec-Butyl, i-Butyl,
- R³: Methyl, Ethyl, n-Propyl, i-Propyl, n-Butyl, sec-Butyl, i-Butyl, n-Pentyl, n-Hexyl.

Bevorzugtes Ausgangsmaterial der Formel II ist Thioacetamid.

Bevorzugtes Ausgangsmaterial der Formel III sind 2-Chlor-4,4,4-trifluoracetessigsäure-alkylester bzw. 2-Chlor-4,4-difluoracetessigsäurealkylester wie 2-Chlor-4,4,4-trifluoracetessigsäuremethyl- oder -ethylester oder 2-Chlor-4,4-difluoracetessigsäuremethyl- oder -ethylester.

Als aprotische Lösungsmittel eignen sich:
N,N-Dialkylamide z.B. N,N-Dimethylformamid, N,N-Diethylformamid, N,N-Dimethylacetamid,
cyclische Harnstoffe z.B. N,N'-Diemthyl-ethylenharnstoff, N,N'-Diemthyl-propylenharnstoff,
Lactame z.B. N-Methylpyrrolidon, N-Methylpiperidon,
Nitrile z.B. Acetonitril, Propionitril, Benzonitril,
halogenierte Kohlenwasserstoffe, die unter den Reaktionsbedingungen inert sind, z.B. Dichlormethan, Chloroform,
Ketone z. B. Aceton, Methylethylketon, Methylisobutylketon,
Ether z. B. 1,2-Dimethoxiethan, Tetrahydrofuran, Dioxan
oder Mischungen dieser Verbindungen.

Als wasserabspaltende Mittel eignen sich z.B.

Anhydride z. B. Acetanhydrid, Trichloracetanhydrid, Chloressigsäureanhydrid, Trifluoressigsäureanhydrid, Perfluorbuttersäureanhydrid,
Carbonsäurechloride z. B. Acetylchlorid, Trichloracetylchlorid,
Chlorderivate der Kohlensäure wie z. B. Phosgen, Chlorkohlensäuretrichlormethylester, Chlorkohlensäure-methyl oder -ethylester,
Sulfonylchloride z. B. Methansulfonylchlorid, Trichlormethylsulfonylchlorid, Trifluormethylsulfonylchlorid, Benzolsulfonylchlorid, Tosylchlorid,
Chlorverbindungen der schwefligen Säure, Schwefelsäure, phosphorigen Säure oder Phosphorsäure z. B. Thionylchlorid, Sulfurylchlorid, Chlorsulfonsäure, Phosphortrichlorid, Phosphoroxitrichlorid.

Insbesondere in Kombination mit Chlorverbindungen der schwefligen Säure oder Schwefelsäure ist die Verwendung von Nitrilen, z.B. Acetonitril als Lösungsmittel oder als Bestandteil von Lösungsmittelgemischen vorteilhaft.

Als Basen eignen sich im allg. organische Basen z. B. tertiäre Amine z. B. Trimethylamin, Triethylamin, Diisopropyl-ethylamin, Tri-n-Butylamin, N,N-Dimethylcyclohexylamin, N-Methylpyrrolidin, N-Methylpiperidin, Diazabicyclo[2,2,2]octan, Pyridine wie Pyridin, Picoline, Collidin, Chinolin.

Es können aber auch anorganische Basen z. B. Alkalicarbonate, Alkalibicarbonate oder Alkali- oder Erdalkalihydroxide verwendet werden. In diesen Fällen muß vor Zugabe der organischen Base und des wasserabspaltenden Mittels eine Trocknung des aprotischen Lösungsmittels vorgenommen werden.

Die Ausgangsmaterialien der Formel II und III werden vorzugsweise in stöchiometrischen Mengen oder in geringem Überschuß eingesetzt. Das wasserabspaltende Mittel wird in mindestens äquimolarer Menge, insbesondere in äquimolarer Menge oder 1 - 10 %igem molaren Überschuß verwendet.

Die organischen Basen werden in Bezug auf die Ausgangsmaterialien mit mindestens doppelter bis dreifach molarer Menge je nach Art des wasserabspaltenden Mittels eingesetzt, wobei auch Kombinationen von zwei in ihrer Basizität unterschiedliche Basen zweckmäßig sein können. Wird zur Bindung des aus den Reaktionspartnern der Formel II und III gebildeten Chlorwasserstoffs eine anorganische Base benutzt, reduziert sich zwangsläufig die Menge an organischer Base um eine der anorganischen Base äquimolare Menge bei der Behandlung mit dem wasserabspaltenden Mittel.

Die Temperaturen bei der Reaktion der Verbindungen der Formel II und III liegen unter 100°C insbesondere bei -30°C bis 70°C, vorzugsweise bei 0°C bis 40°C. Bei der Anwendung des wasserabspaltenden Mittels liegen die Temperaturen im gleichen Bereich, wobei es zur Beschleunigung der Wasserabspaltung vorteilhaft sein kann, die Reaktion oberhalb von 0°C zu Ende zu führen je nach Art des wasserabspaltenden Mittels.

Die Aufarbeitung des Reaktionsgemisches erfolgt z.B. durch Verdünnen mit Wasser und Extraktion des gebildeten Thiazolcarbonesters mit Lösungsmitteln, die nicht mit Wasser mischbar sind, wie z. B. Hexan, Cyclohexen, Toluol, Ether wie Methyl-tert.-butylether, Dichlormethan oder Gemischen aus diesen Verbindungen.

Bei Verwendung eines aprotischen mit Wasser mischbaren Lösungsmittels für die Durchführung der Reaktion ist es vorteilhaft nach Ende der Reaktion dieses Lösungsmittel zu verdampfen und danach wie vorstehend beschrieben zu verfahren.

Bei Verwendung eines geringen Überschusses an wasserabspaltendem Mittel ist es vorteilhaft, nach der Extraktion des Thiazolcarbonsäureesters eine Wäsche der Extraktionsflüssigkeit, die den Thiazolcarbonsäureester enthält, mit verdünnten Alkalien z.B. Natriumbicarbonat-Lösung zur Entfernung von sauren Bestandteilen anzuschließen. Der nach Verdampfen des Extraktionsmittel erhaltene Thiazolcarbonester der Formel I ist meist sehr rein und kann unmittelbar weiter verarbeitet werden z. B. durch Verseifung zur entsprechenden Säure.

### Beispiel 1

Zu einer Lösung von 15,0 g Thioacetamid in 200 ml Acetonitril tropft man bei 20 bis 25°C 43,7 g 2-Chlor-4,4,4-trifluoracetessigsäureethylester. Nach 12stündigem Rühren bei Raumtemperatur (20°C) kühlt man auf 0°C bis 5°C, tropft eine Mischung aus 20,2 g Triethylamin und 44,6g 2-Picolin zu, rührt 15 Minuten nach, fügt danach 44,1 g Trifluoracetanhydrid tropfenweise zu und rührt eine Stunde bei Raumtemperatur nach. Nach dem Verdampfen des Lösungsmittel im Vakuum (20 mbar, 35°C) wird der Rückstand zwischen 200 ml Methyl-tert.-butylether und 200 ml 2 n Salzsäure verteilt. Nach Waschen der organischen Phase mit 200 ml Wasser, 100 ml Natriumbicarbonat-Lösung, Trocknen und Verdampfen des Lösungsmittels isoliert man 45,4 g (95 %) 2-Methyl-4-trifluormethyl-thiazolcarbonsäureethylester. Reinheit HPLC (Hochdruckflüssigkeitschromatographie) (Acetonitril/Wasser 7:3) 90 %.

### Beispiel 2

Zu einer Lösung von 3,0 g Thioacetamid in 40 ml Acetonitril tropft man bei 5°C 8,7 g 2-Chlor-4,4,4-trifluoracetessigsäureethylester. Nach 12stündigem Rühren bei Raumtemperatur (20°C) kühlt man auf 5°C, tropft eine Mischung von 8,9 g Triethylamin und 4,8 g 2-Picolin zu, rührt 10 Minuten nach und fügt dann tropfenweise 5,3 g Methansulfonylchlorid zu und rührt 6 Stunden bei Raumtemperatur nach. Nach Verdampfen des Lösungsmittels wird der Rückstand zwischen 40 ml Methyl-tert.-butyläther und 40 ml 2 n Salzsäure verteilt. Nach Waschen der organischen Phase mit 40 ml Natriumbicarbonat-Lösung, Trocknen und Verdampfen des Lösungsmittels isoliert man 8,7 g (91 %) 2-Methyl-4-trifluormethyl-thiazolcarbonsäureethylester. Reinheit HPLC (Acetonitril/Wasser 7:3) 96,4 %.

### Beispiel 3

Zu einer Lösung von 16,1 g Thioacetamid in 180 ml Acetonitril tropft man bei 5°C bis 10°C 47,0 g 2-Chlor-4,4,4-trifluoracetessigsäureethylester. Nach 10stündigem Rühren bei Raumtemperatur kühlt man auf 3 bis 7°C, tropft 71,6 g Triethylamin und danach 28,1 g Thionylchlorid zu und rührt eine Stunde bei Raumtemperatur nach. Nach Verdampfen des Lösungsmittels wird der Rückstand zwischen 200 ml Wasser und 100ml Methyl-tert.-butyläther erteilt. Nach Extraktion der wäßrigen Phase mit 100 ml Methyl-tert.-butylether werden die vereinigten organischen Phasen mit 20 ml Wasser gewaschen. Nach Trocknen und Verdampfen des Lösungsmittels erhält man 48,7 g (94,7 %) 2-Methyl-4trifluormethylthiazol-carbonsäureethylester. Reinheit HPLC (Acetonitril/Wasser 7:3) 98 %.

## Patentansprüche

1. Verfahren zur Herstellung von 2-Alkyl-4-fluormethylthiazolcarbonsäurealkylestern, der Formel I 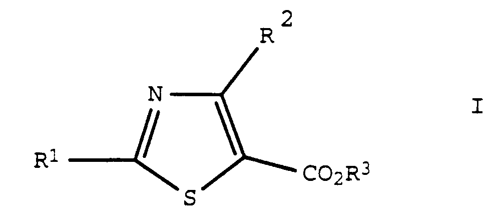 in welcher
R¹ C₁-C₄-Alkyl
R² Difluormethyl oder Trifluormethyl und
R³ C₁-C₆-Alkyl
bedeuten,
dadurch gekennzeichnet, daß man in 4-Stellung durch Fluor substituierte 2-Chloracetessigsäure-alkylester der Formel III 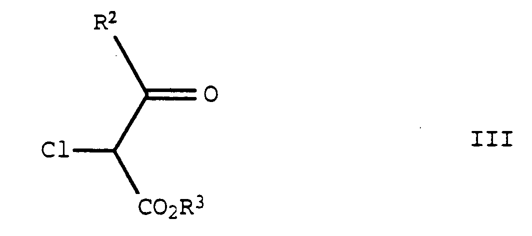 in der R² und R³ die oben angegebenen Bedeutungen haben, mit Carbonsäurethioamiden der Formel II 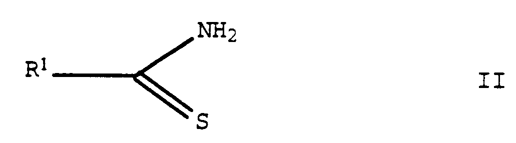 in welcher R¹ die oben angegebene Bedeutung hat,
in einem aprotischen Lösungsmittel bei Temperaturen unter 100°C zur Reaktion bringt und mit einem wasserabspaltenden Mittel, gegebenenfalls in Gegenwart von mindestens doppelten bis dreifachen molaren Mengen von Basen, bei Temperaturen unter 80°C umsetzt.

2. Verfahren zur Herstellung von 2-Methyl-4-trifluormethylthiazolcarbonsäure-alkylestern gemäß Anspruch 1, dadurch gekennzeichnet, daß man einen 2-Chlor-4,4,4-trifluoracetessigsäure-alkylester mit Thioacetamid umsetzt.

3. Verfahren zur Herstellung von 2-Methyl-4-difluormethylthiazolcarbonsäure-alkylestern gemäß Anspruch 1, dadurch gekennzeichnet, daß man einen 2-Chlor-4,4-difluoracetessigsäure-alkylester mit Thioacetamid umsetzt.

4. Verfahren nach Anspruch 1, dadurch gekennzeichnet, daß man die Reaktion der Verbindungen II und III bei Temperaturen von 0 bis 40°C durchführt.

5. Verfahren nach Anspruch 1, dadurch gekennzeichnet, daß man die Umsetzung mit dem wasserabspaltenden Mittel bei Temperaturen von 0 bis 40°C durchführt.

6. Verfahren nach Anspruch 1, dadurch gekennzeichnet, daß man als Verbindung der Formel II Thioacetamid verwendet.

7. Verfahren nach Anspruch 1, dadurch gekennzeichnet, daß man als Verbindung der Formel III 2-Chlor-4,4,4-trifluoracetessigsäureethylester verwendet.

8. Verfahren nach Anspruch 1, dadurch gekennzeichnet, daß man als aprotisches Lösungsmittel Acetonitril oder Acetonitril enthaltende Lösungsmittelgemische verwendet.

## Claims

1. A process for preparing alkyl 2-alkyl-4-fluoromethylthiazolecarboxylates of the formula I 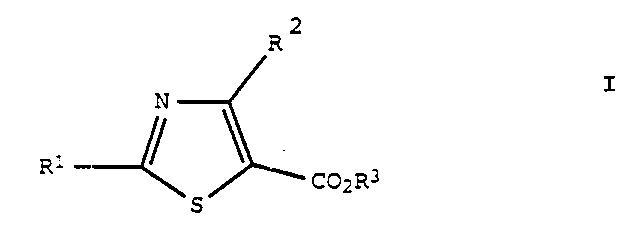 where
R¹ is C₁-C₄-alkyl,
R² is difluoromethyl or trifluoromethyl and
R³ is C₁-C₆-alkyl,
which comprises reacting alkyl 2-chloroacetoacetates, substituted in the 4-position by fluorine, of the formula III 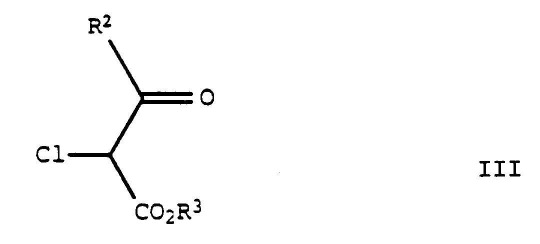 where R² and R³ have the abovementioned meanings,
with thiocarboxamides of the formula II 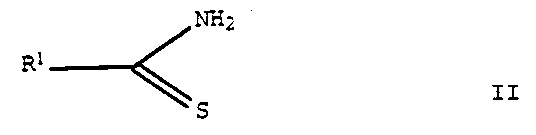 where R¹ has the abovementioned meaning,
in an aprotic solvent at below 100°C and reacting with a dehydrating agent, if appropriate in the presence of at least two to three times the molar amounts of bases, at below 80°C.

2. A process for preparing alkyl 2-methyl-4-trifluoromethylthiazolecarboxylates as claimed in claim 1, wherein an alkyl 2-chloro-4,4,4-trifluoroacetoacetate is reacted with thioacetamide.

3. A process for preparing alkyl 2-methyl-4-difluoromethylthiazolecarboxylates as claimed in claim 1, wherein an alkyl 2-chloro-4,4-difluoroacetoacetate is reacted with thioacetamide.

4. The process as claimed in claim 1, wherein the reaction of the compounds II and III is carried out at from 0 to 40°C.

5. The process as claimed in claim 1, wherein the reaction with the dehydrating agent is carried out at from 0 to 40°C.

6. The process as claimed in claim 1, wherein the compound of the formula II used is thioacetamide.

7. The process as claimed in claim 1, wherein the compound of the formula III used is ethyl 2-chloro-4,4,4-trifluoroacetoacetate.

8. The process as claimed in claim 1, wherein the aprotic solvent used is acetonitrile or acetonitrile-containing solvent mixtures.

## Revendications

1. Procédé de préparation de 2-alkyl-4-fluorométhyl-thiazol carboxylate d'alkyle de formule I 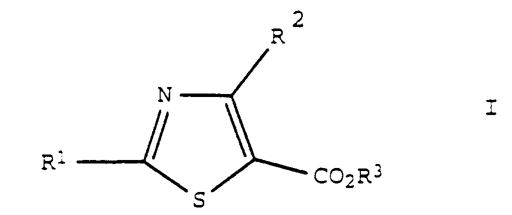 dans laquelle
R¹ représente alkyle en C1-C4
R², difluorométhyle ou trifluorométhyle et
R³, alkyle en C1-C6
caractérisé par le fait que l'on porte en réaction, à des températures inférieures à 100°C, dans un solvant aprotique du 2-chloroacétylacétate d'alkyle substitué par fluor en position 4, de la formule III 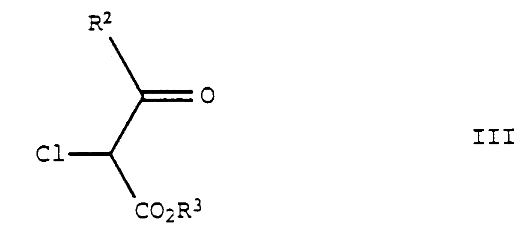 dans laquelle R² et R³ ont les significations sus-indiquées avec des éthioamides d'acide carboxylique de formule II 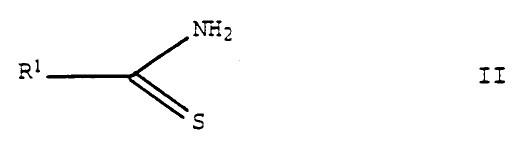 dans laquelle R¹ a la signification sus-indiquée et on fait agir à des températures inférieures à 80°C avec un milieu déshydratant, éventuellement en présence de quantités molaires au moins doubles à triples de bases.

2. Procédé de préparation de 2-méthyl-4-trifluorométhylthiazol carboxylate d'alkyle selon la revendication 1, caractérisé par le fait que l'on fait réagir un 2-chloro-4,4,4-trifluoroacétylacétate d'alkyle avec du thioacétamide.

3. Procédé de préparation de 2-méthyl-4-difluorométhylthiazolcarboxylate d'alkyle selon la revendication 1, caractérisé par le fait que l'on fait réagir un 2-chloro-4,4-difluoroacétylacétate d'alkyle avec du thioacétamide.

4. Procédé selon la revendication 1, caractérisé par le fait que l'on effectue la réaction des composés II et III à des températures de 0 à 40°C.

5. Procédé selon la revendication 1, caractérisé par le fait que l'on effectue la réaction avec le milieu déshydratant à des températures de 0 à 40°C.

6. Procédé selon la revendication 1, caractérisé par le fait que, comme composé de formule II, on utilise du thioacétamide.

7. Procédé selon la revendication 1, caractérisé par le fait que, comme composé de formule III, on utilise du 2-chloro-4,4,4-trifluoroacétylacétate d'éthyle.

8. Procédé selon la revendication 1, caractérisé par le fait que, comme solvant aprotique, on utilise de l'acétonitrile ou un mélange de solvant contenant de l'acétonitrile.
